# EUROPEAN PATENT APPLICATION

(11) **EP 2 703 413 A1**
(43) Date of publication of application: **05.03.2014**
(21) Application number: 13182165.4
(22) Date of filing: 29.08.2013
(51) Int. Cl.: C07K 14/78, C12N 15/82, C12N 15/85, A61L 27/36

(54) **Recombinant vector, transgenic fish egg using the same and biomaterial using the same**

(30) Priority: 30.08.2012 US 201261694877 P
(71) Applicant: Body Organ Biomedical Corp., Taipei 10658 (TW)
(72) Inventor: Ken, Chuian-Fu, 10658 Taipei City (TW); Gong, Hong-Yi, 10658 Taipei City (TW); Wu, Jen-Leih, 10658 Taipei City (TW); Lai, Horng-Ji, 10658 Taipei City (TW); Lin, Chien-Cheng, 10658 Taipei City (TW); Chen, Yong-Guei, 10658 Taipei City (TW); Chou, Cheng-Hung, 10658 Taipei City (TW)
(74) Representative: Viering, Jentschura & Partner Patent- und Rechtsanwälte

(57) **Abstract**

A recombinant vector, transgenic fish egg using the same and biomaterial using the same are applied to provide a transgenic fish that secreting recombinant human procollagens or collagens, and further to provide the biomaterial having the recombinant human procollagens or collagens and extract the recombinant human procollagens or collagens from the part(s), having the recombinant human procollagens or collagens, of the transgenic fish.

## Description

### BACKGROUND

### Technical Field

The invention relates to a manufacturing technology of a biomedical material, and particularly to a recombinant vector, a transgenic fish using the same and a biomaterial using the same.

### Related Art

Collagens are important reconstruction materials in biomedical material applications, and have the capabilities of improving tissue growing, improving wound healing, using as plastic materials, using as wound dressing for bums, and etc.. Additionally, the market scale of collagens continues to expand. Consequently, development of collagens and derivatives thereof with higher medical values and wider diversities is a topic for researchers to resolve.

So far, the expression system for producing recombinant collagens includes Escherichia coli (*E*. *coli*), yeast (such as Saccharomyces cerevisiae), mammalian cell lines, insect cell lines, transgenic animals and transgenic plants. The post-translational modification cannot be achieved in the *E*. *coli* expression system. The yeast expression system lacks of the activity of pmlyl-4-hygroxylase (PH), while the yeast expression system where the yeasts grown on Methanol has the best yields of the collagens among these expression systems. The mammalian cell expression system has low yields of the collagens and is limited to use particular cells. In the insect cell expression system, there is low activity of pmlyl-4-hygroxylase. For the transgenic animals (such as silk worms and mice), and transgenic plants (such as tobacco), the collagen products are over cross-linking.

Until now, baculovirus expression system with human pmlyl-4-hygroxylase and collagen genes has been established (referring to US Patent No. 5077214 and US Patent No. 5593859). However, the transfected insect cells will die within 72 hours after transfection, so that the recombinant collagens are produced temporarily. In addition, due to the cell lysis, the recombinant collagens are difficult to recycle or purify hardly. Furthermore, the damages of endoplasmic reticulum and Golgi bodies by the baculovirus further restrict collagen production.

### SUMMARY

In one embodiment, a biomaterial includes a recombinant human procollagen or collagen obtained from and secreted by a fish.

In some embodiment, the biomaterial further includes at least one fish scale of the fish, and each fish scale has the recombinant human procollagen or collagen.

In some embodiment, the recombinant human procollagen or collagen is expressed by at least one human procollagen or collagen gene in the fish.

In another embodiment, a transgenic fish egg or embryo includes a fish chromosome and a recombinant vector. The recombinant vector is inserted in the fish chromosome, and the recombinant vector has a human procollagen or collagen gene.

In yet another embodiment, a recombinant vector includes an upstream gene and a human procollagen or collagen gene. The upstream gene is constructed from a fish, and the human procollagen or collagen gene is connected to downstream of the upstream gene.

In some embodiments, the upstream gene is a promoter already existing in the fish, an enhancer already existing in the fish, or the combination thereof.

In some embodiments, the recombinant vector further includes a report gene, and the report gene is connected to downstream of the human procollagen or collagen gene.

As above, the recombinant vector, the transgenic fish using the same and the biomaterial using the same according to the invention can be applied to provide a transgenic fish that secrets the recombinant human procollagens or collagens in part(s) of the transgenic fish, and further the part(s) of the transgenic fish that secrets the recombinant human procollagens or collagens can be treated as the biomaterials and/or can be treated to purify the recombinant human procollagens or collagens.

The detailed features and advantages of the disclosure are described below in great detail through the following embodiments, the content of the detailed description is sufficient for those skilled in the art to understand the technical content of the disclosure and to implement the disclosure there accordingly. Based upon the content of the specification, the claims, and the drawings, those skilled in the art can easily understand the relevant objectives and advantages of the disclosure.

### BRIEF DESCRIPTION OF THE DRAWINGS

The disclosure will become more fully understood from the detailed description given herein below for illustration only and thus not limitative of the disclosure, wherein:
FIG. 1 is a flow chart of one embodiment of a transfect method for a transgenic fish egg;
FIG. 2 is a schematic view of one embodiment of a recombinant vector;
FIG. 3 is a detailed flow chart of one embodiment of step S 10 shown in FIG. 1;
FIG. 4 is a detailed flow chart of one embodiment of step S110 shown in FIG. 3;
FIG. 5 is a detailed flow chart of one embodiment of step S120 shown in FIG. 3;
FIG. 6 is a detailed flow chart of one embodiment of step S130 shown in FIG. 3;
FIG. 7 is a detailed flow chart of one embodiment of step S 140 shown in FIG. 3; and
FIG. 8 is a schematic view of one embodiment of a recombinant plasmid having human type I, alpha 1 procollagen or collagen gene.

### DETAILED DESCRIPTION

The ordinal terms, such as first, second, third, and etc., mentioned hereafter are applied to distinguish the elements only, but not applied to sequence or to restrict the elements; also they are not applied to limit the scope of the disclosure.

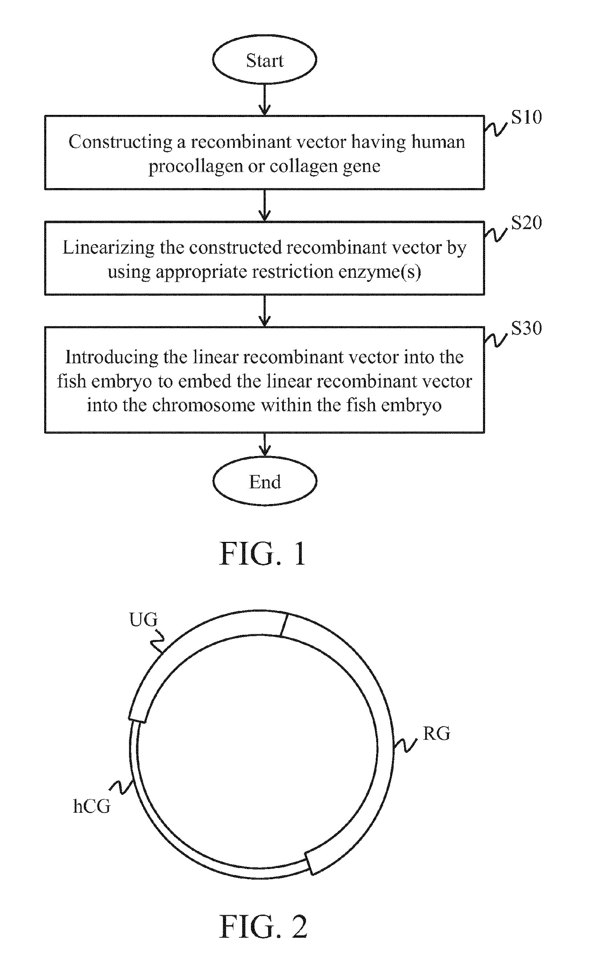

Please refer to FIG. 1, in which a method for preparing a transgenic fish egg or embryo includes constructing a recombinant vector having a human procollagen or collagen gene hCG (step S10), linearizing the constructed recombinant vector by using appropriate restriction enzyme(s) (step S20) and introducing the linear recombinant vector into the fish embryo to embed the linear recombinant vector into the chromosome within the fish embryo (step S30).

Please refer to FIG. 2, in which the recombinant vector includes an upstream gene UG and the human procollagen or collagen gene hCG, and the human procollagen or collagen gene hCG is connected to downstream of the upstream gene UG. The upstream gene UG at least has a promoter which specifically drives the expression of downstream generic sequence (that is, human procollagen or collagen gene hCG) in a germ cell (that is, embryo), an enhancer co-working with the promoter, or the combination thereof. The promoter is present in the fish and has tissue-specific. The enhancer is present in the fish.

In some embodiments, the promoter can be, for example, keratin 4 (K4) promoter, keratin 5 promoter or krtt1c6 promoter.

Wherein, the sequence of the keratin 4 promoter is shown as SEQ ID NO: 1, and the sequence of the krtt1c6 promoter is shown as SEQ ID NO: 2.

In some embodiments, the human procollagen or collagen gene hCG can be a single-stranded amino acid sequence or a triple-stranded amino acid sequence.

The human procollagen or collagen gene hCG can be one of the human type I-XXVIII procollagens or collagens or the combination formed with at least two of the human type I-XXVIII procollagens or collagens. The human procollagen or collagen gene hCG can be single-stranded or multi-stranded. For example, the human type I procollagen or collagen has two α1 chains, and one α2 chain, and the human type II procollagen or collagen has threeα1 chains. As the matched types of the human procollagen or collagen are well known by those skilled in the art, additional details relating to the matched types of the human procollagen or collagen are not provided.

The sequence of the human type I procollagen or collagen gene is shown as SEQ ID NO: 3 and 4, and the sequence of the human type II procollagen or collagen gene is shown as SEQ ID NO: 5. Here, the gene sequence of each chain of the human type I and II procollagen or collagens is exemplarily shown in sequence listing, but not limited thereto.

In some embodiments, the recombinant vector can further have a report gene RG, and the report gene RG is connected to downstream of the human procollagen or collagen gene hCG.

The report gene RG can be, for example, a luciferase gene, a fluorescent protein gene, a lac Z gene (aβ-galactosidase gene) or etc..

The fluorescent protein gene can be the green fluorescent protein (GFP) gene, the modified green fluorescent protein gene, the enhanced green fluorescent protein (EGFP) gene, the red fluorescent protein (RFP) gene, the enhanced red fluorescent protein (ERFP) gene, the blue fluorescent protein (BFP) gene, the enhanced blue fluorescent protein (EBFP) gene, the yellow fluorescent protein (YFP) gene, the enhanced yellow fluorescent protein (EYFP) gene, the cyan fluorescent protein (CFP) gene, the enhanced cyan fluorescent protein (ECFP) gene, or etc..

In some embodiments, the recombinant vector can be a recombinant plasmid, a recombinant viral vector, a cosmid, or an artificial chromosome.

In some embodiments, the introducing step (the step S30) can be implemented by microinjection, transfection (such as DEAE-dextran precipitation or calcium phosphate precipitation), being infected with viral vectors (such as retrovirus or adenovirus), electroporation, increasing the cell membrane permeability, gene gun, liposome delivery (such as Lipofectin®, Lipofectamine®, Superfect®, Transfectam® or other liposome developed according to conventional methods), receptor mediated absorption, other pinocytosis mechanisms or etc..

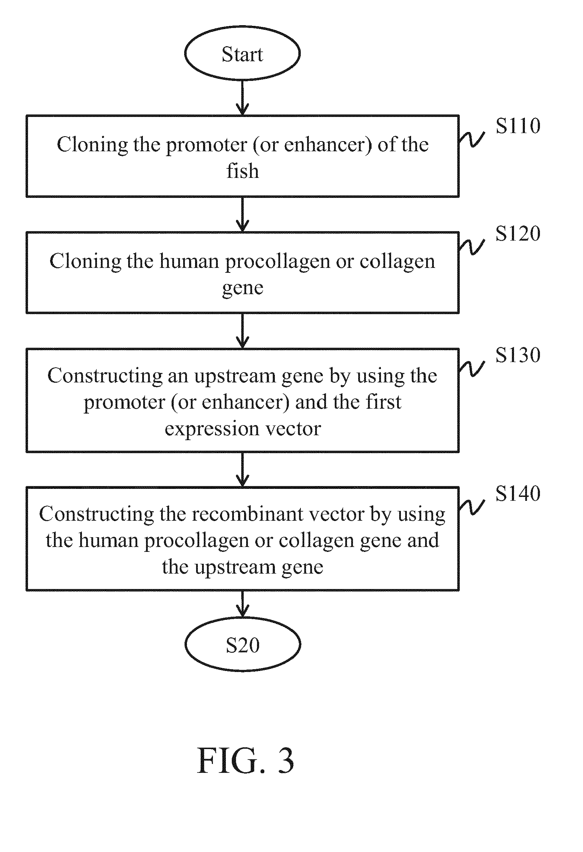

Please refer to FIG. 3, in which in some embodiments, the step S10 includes cloning the promoter (or/and the enhancer) of the fish (step S110), cloning the human procollagen or collagen gene hCG (step S120), constructing the upstream gene UG by using the promoter (or/and the enhancer) and the first expression vector EV (step S130) and constructing the recombinant vector having the human procollagen or collagen gene hCG and the upstream gene UG (step S 140).

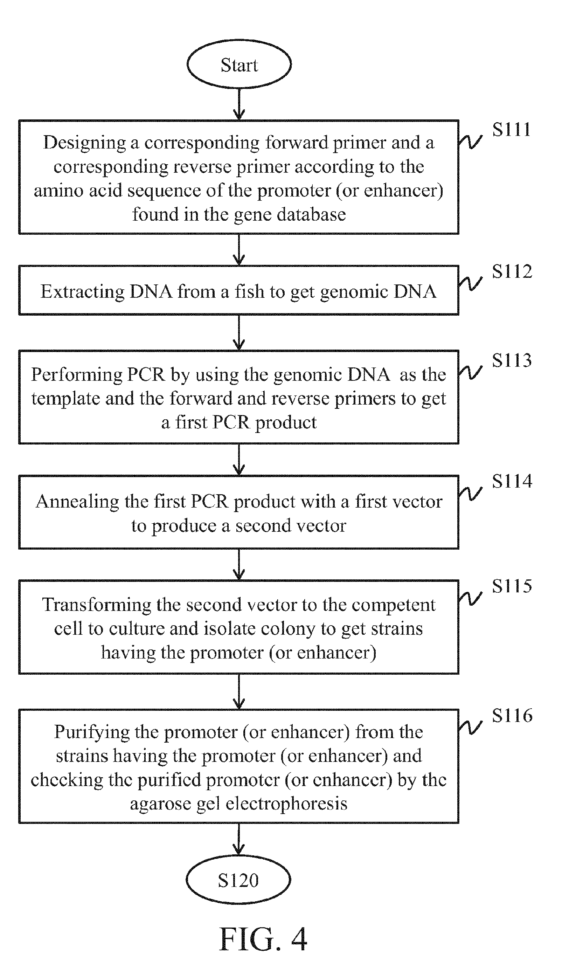

Please refer to FIG. 4, in which in some embodiments, the step S110 includes designing a corresponding forward primer (5' primer), and a corresponding reverse primer (3' primer), according to the amino acid sequence of the promoter (or the enhancer) found in the gene database, extracting DNA (deoxyribonucleic acid), from a fish (fish tissue), to obtain genomic DNA (step S112), performing the polymerase chain reaction (PCR) by using the genomic DNA got in step S112 as the template and the forward and reverse primers got in step S111 to react with the reagents, such as the DNA polymerase, deoxyribonucleoside triphsphate (dNTP), and etc., to got a first PCR product (step S113), annealing the first PCR product with a first vector to produce a second vector (step S114), transforming the second vector to the competent cell to culture and isolate colony to obtain strains having the promoter (or the enhancer) (step S115), and purifying the promoter (or the enhancer) from the strains having the promoter (or the enhancer) and checking the purified promoter (or the enhancer) by the agarose gel electrophoresis (step S116).

In some embodiments, the fish can be a Zebra fish, etc. As for the sequence of the SEQ ID NO: 1, for example, the forward primer and the reverse primer can be the sequences shown as SEQ ID NO: 6 and SEQ ID NO: 7, respectively.

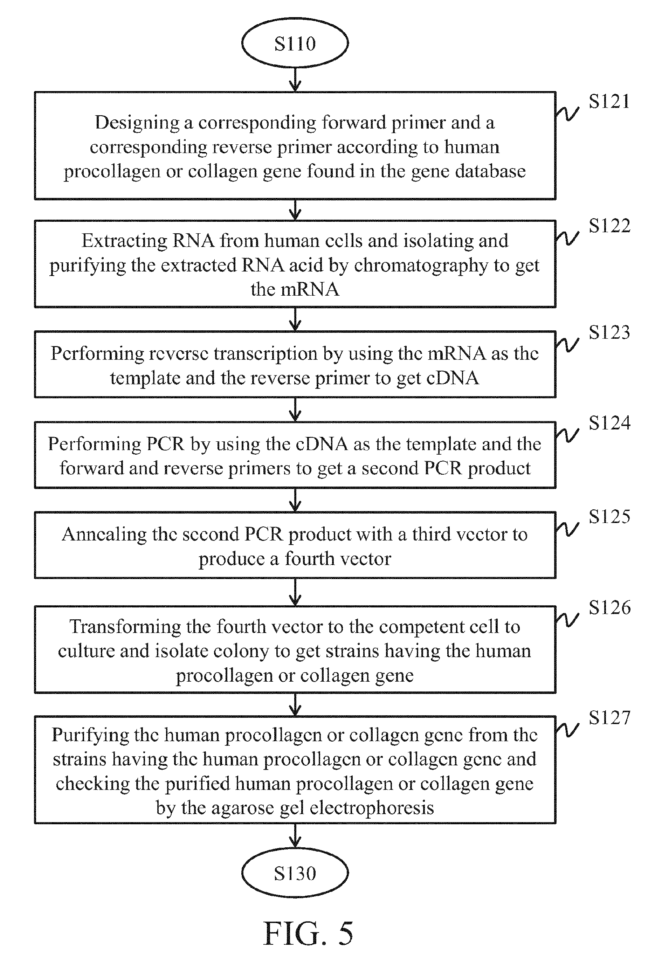

Please refer to FIG. 5, in which in some embodiments, the step S 120 includes designing a corresponding forward primer and a corresponding reverse primer according to the message RNA (mRNA) of the human procollagen or collagen gene hCG found in the gene database (step S121), extracting ribonucleic acid (RNA) from human cells and isolating and purifying the extracted RNA by chromatography to obtain the mRNA (step S122), performing reverse transcription (RT) by using the message RNA got in step S122 as the template and the reverse primer get in step S121 as the primer to obtain complementary DNA (cDNA) (step S123), performing the PCR by using the cDNA got in step S123 as the template and the forward and the reverse primers get in step S121 as the primers to react with the reagents, such as the DNA polymerase, deoxyribonucleoside triphsphate and etc., to obtain a second PCR product (step S124), annealing the second PCR product with a third vector to produce a fourth vector (step S125), transforming the fourth vector to the competent cell to culture and isolate colony to obtain strains having the human procollagen or collagen gene hCG (step S126), and purifying the human procollagen or collagen gene hCG from the strains having the human procollagen or collagen gene hCG and checking the purified human procollagen or collagen gene hCG by the agarose gel electrophoresis (step S127).

In some embodiments, the human cell can be the firoblast, the chondrocyte, etc. As for the sequence of the SEQ ID NO: 3, for example, the forward primer can be the sequences shown as SEQ ID NO: 8 and SEQ ID NO: 10, and the reverse primer can be the sequences shown as SEQ ID NO: 9 and SEQ ID NO: 11. As for the sequence of the SEQ ID NO: 4, for example, the forward primer and the reverse primer can be the sequences shown as SEQ ID NO: 12 and SEQ ID NO: 13, respectively. As for the sequence of the SEQ ID NO: 5, for example, the forward primer can be the sequences shown as SEQ ID NO: 14 and SEQ ID NO: 16 and the reverse primer can be the sequences shown as SEQ ID NO: 15 and SEQ IN NO: 17.

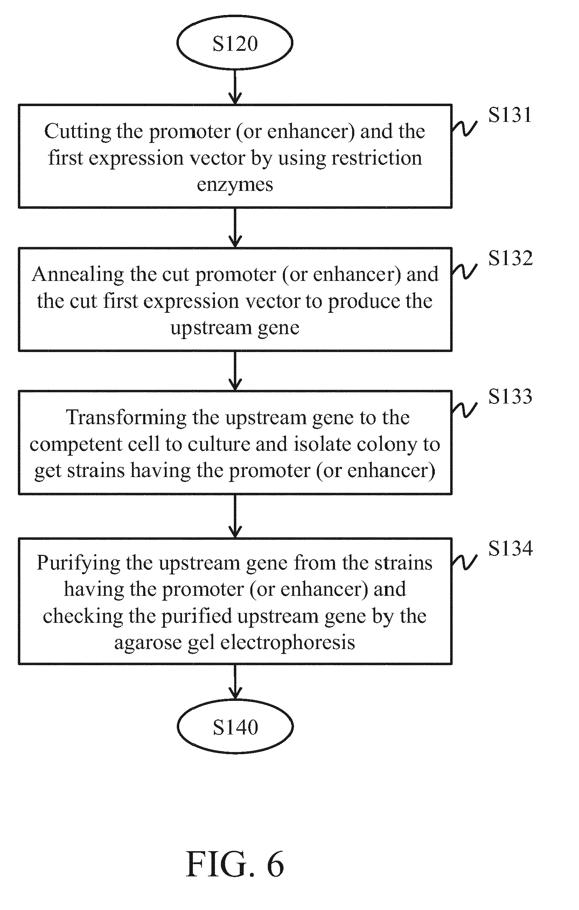

Please refer to FIG. 6, in which in some embodiments, the step S130 includes individually cutting the promoter (or the enhancer) got in step S116 and the first expression vector by using one or two restriction enzymes (step S 131), annealing the cut promoter (or the enhancer) and the cut first expression vector EV to produce the upstream gene UG (step S132), transforming the upstream gene UG to the competent cell to culture and isolate colony to obtain strains having promoter (or the enhancer) (step S133), and purifying the upstream gene UG from the strains having promoter (or the enhancer) and checking the purified upstream gene UG by the agarose gel electrophoresis (step S134).

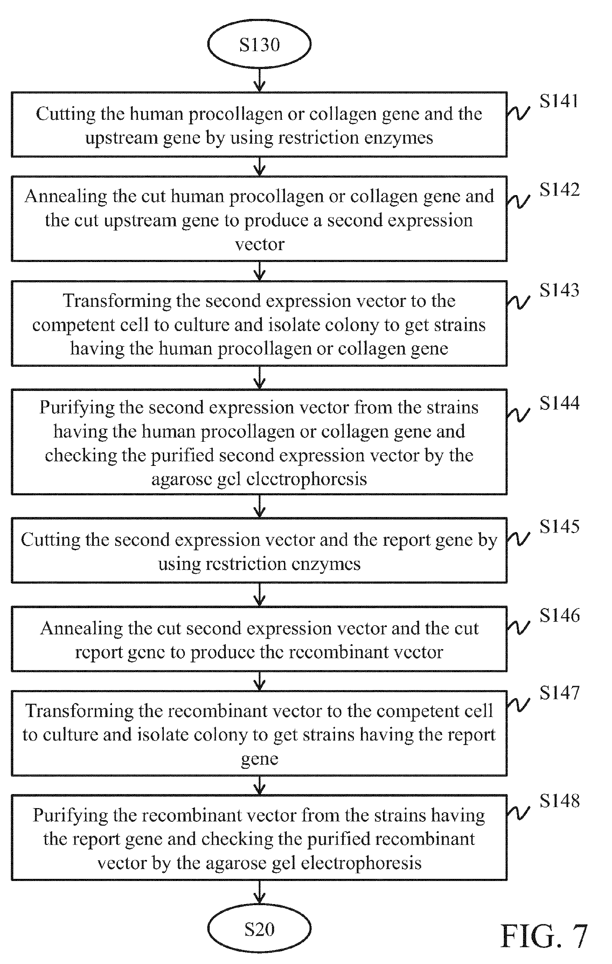

Please refer to FIG. 7, in which in some embodiments, the step S 140 includes individually cutting the human procollagen or collagen gene hCG got in step S127 and the upstream gene UG got in step S134 by using one or two restriction enzymes (step S141), annealing the cut human procollagen or collagen gene hCG and the cut upstream gene UG to produce a second expression vector (step S 142), transforming the second expression vector to the competent cell to culture and isolate colony to obtain strains having the human procollagen or collagen gene hCG (step S 143), purifying the second expression vector from the strains having the human procollagen or collagen gene hCG and checking the purified second expression vector by the agarose gel electrophoresis (step S144), cutting the second expression vector got in step S144 and the report gene RG by using one or two restriction enzymes (step S 145), annealing the cut second expression vector and the cut report gene RG to produce the recombinant vector (step S146), transforming the recombinant vector to the competent cell to culture and isolate colony selection to obtain strains having the report gene RG (step S147), and purifying the recombinant vector from the strains having the report gene and checking the purified recombinant vector by the agarose gel electrophoresis (step S 148).

In some embodiments, when it is designed that the recombinant vector does not have the report gene RG, the steps from S145 to S148 can be omitted, and at this time, the second expression vector got in step S 144 is the recombinant vector.

Here, as the proper competent cells are known by those skilled in the arts, and many competent cells are commercially available, such as prakaryotic cell, eucaryotic cell or etc, therefore, further details about the competent cells are not provided.

As should known by those skilled in the arts, the transgenic fish egg is the embryo in which the aforementioned recombinant vector is artificially introduced, or is a fish egg spawned by a fish grown from the transgenic embryo. For convenience, the embryo in which the aforementioned recombinant vector is artificially introduced and the coming generations thereof are generally called as the transgenic fish hereafter. That is, the cell of the transgenic fish has at least one clone body of the recombinant vector. For convenience, the recombinant vector artificially introduced and the clone bodies thereof are generally called as the recombinant gene structure hereafter.

Consequently, according to the types of the upstream gene UG selected, the upstream gene UG in the clone body can directly or indirectly drive the human procollagen or collagen gene hCG in the clone body to secret the recombinant human procollagen or collagen in the fish scales, the fish skins, the fish eyes or other part(s) of the transgenic fish. Further, the part(s) of the transgenic fish where the recombinant human procollagen or collagen is secreted can treated as the biomaterials (e.g. the biomaterial having the secreted recombinant human procollagens or collagens and inorganic substances) and/or can be treated to purify the recombinant human procollagen or collagen.

Here, each secreted recombinant human procollagen can be one of type I-XXVIII procollagens. Additionally, each secreted recombinant human collagen can be one of the type I-XXVIII collagens or the combination formed with at least two of the type I-XXVIII collagens.. Further, all the three chains of the secreted recombinant human collagen can be oriented from the human procollagen or collagen gene hCG. Alternatively one or two of the three chains of the secreted recombinant human procollagen or collagen are oriented from the human procollagen or collagen gene hCG and rest chain(s) thereof are oriented from fish procollagen or collagen gene.

In some embodiments, the part(s) of the transgenic fish having the recombinant human procollagens or collagens can be fish scales, fish skins, fish eyes or the combination thereof.

Hereafter, some practical experiments are described as detailed explanations about the disclosure, but not limited thereto. In experiments, the DNA sequencing is done by Tri-I Biotech, Inc., and the used DNA analyzers system is ABI 3730 Genetic Analyzer. The LB (Luria-Bertaini) medium is prepared by dissolving 20g LB powder (Bio Basic Inc.) in dH2O, and then adding dH2O to total volume of 1L. The prepared LB medium is sterilized in autoclave. The LB powder includes 10g Trypton, 5g Yeast extract and 5g NaCl (Sodium chloride). LB/Amp (LB plate with ampicillin) medium is prepared by adding 50 µg/mL ampicillin into LB medium.

### First experiment: Extracting DNA from a fish

The genomic DNA is extracted from the wild-type zebrafish (Danio rerio AB strain) by a GenoMaker kit. The GenoMaker kit includes GenoMaker reagent, RNase cocktail and Ethanol precipitate Buffer.

Firstly, the zebrafish tissue is ground by the liquid nitrogen grinding technique or by the homogenizer. Next, 1 ml GenoMaker reagent is added to about 10 mg to 100 mg ground fish tissue to lysis the cells, to obtain the lysis homogeneous sample. Then, the lysis homogeneous sample (1 ml) is incubated in room temperature for 3 to 5 minutes and 500 µl phenol/chloroform (v:v = 1:1) is added to and mixed uniformly with the lysis homogenous sample to obtain a first mixed solution. The first mixed solution is centrifuged at 12000 rpm for 5 minutes, and the supernatant liquid of the first mixed solution is carefully absorbed and transferred to a new centrifuge tube. Further, 0.7 to 0.8 ml isopropanol is added to the centrifuge tube and uniformly mixed with the supernatant liquid of the first mixed solution to obtain a second mixed solution. The second mixed solution is centrifuged at 12000 rpm for 5 minutes, and the supernatant liquid of the second mixed solution is removed and the precipitates are left in centrifuge tube. 40 µl RNase cocktail is added to the centrifuge tube having precipitates and incubated in 37°C for 5 minutes to dissolve the subhumid precipitates. Next, 600µl Ethanol precipitate Buffer is added to the centrifuge tube and uniformly mixed with the precipitate solution to obtain a third mixed solution. The third mixed solution is centrifuged at 12000 rpm for 5 minutes, and the supernatant liquid is removed and the precipitates are left in the centrifuge tube. Finally, 50 to 200 µl TE buffer is added to the centrifuge tube to dissolve the precipitates and then the genomic DNA of the fish is acquired. The genomic DNA is stored in -20°C for further experiments.

### Second experiment: Cloning the promoter of the fish

### Polymerase chain reaction:

K4 promoter shown as SEQ ID NO: 1 is taken as an example. Here, the sequence of SEQ ID NO: 6 is applied as the forward primer, and the sequence of SEQ ID NO: 7 is applied as the reverse primer.

According to Table 1, reagents are added into a 0.2 ml micro test tube to perform PCR to obtain the first PCR product. Here, the first stage condition of PCR is reacting at 94°C for 1 minute; the second stage condition is reacting at 58°C for 1 minute; the third stage condition is reacting at 72°C for 10 seconds, and each stage is progressed in order for 3S cycles.

Then, the first PCR product is checked by 1% agarose gel electrophoresis to ensure the K4 promoter is present in the first PCR product or not.

**Table 1**

| Reagent | Usage amount |
|---|---|
| Template DNA (10-times diluted Genomic DNA of the fish) | 2 µl |
| 5' primer (3pmol/µl) | 3 µl |
| 3' primer (3pmol/µl) | 3 µl |
| 10X Taq DNA polymerase buffer | 5 µl |
| Taq DNA polymerase (SuperTag™XL) | 0.5 µl |
| 2.5mM dNTP | 4 µl |
| Sterile H₂O | 32.5 µl |
| Total amount | 50 µl |

### Annealing reaction:

According to Table 2, reagents are added into a 0.2 ml micro test tube to perform the annealing reaction (4°C, overnight), to obtain the fourth mixed solution (which contains the second vector).

**Table 2**

| Reagent | Usage amount |
|---|---|
| First PCR product | 4 µL |
| yT&A vector (first vector) | 1 µL |
| T4 DNA ligase buffer (A) | 1 µL |
| T4 DNA ligase buffer (B) | 1 µL |
| T4 DNA ligase | 1 µL |
| Sterile H₂O | 2 µL |
| Total amount | 10 µL |

### Transformation reaction:

Here, the used competent cells are the ECOS 101 (*E*. *coli*) strains.
10 µl fourth mixed solution is added into and mixed uniformly with 100 µl competent cell to produce a first bacterial solution. After mixing, immediately put the first bacterial solution into an ice bath for about 5 minutes, and followed by putting the first bacterial solution into 42°C for 45 seconds to proceed the heat-shock reaction. After the heat-shock reaction, the first bacterial solution is put into the ice bath again for 5 minutes. Lastly, the ice bathed first bacterial solution is uniformly coated in the LB/Amp medium, to incubate at 37°C overnight.

### Colony selection:

The colony grown in the aforementioned LB/Amp medium is re-cultured in a new LB/Amp medium and incubated at 37°C overnight.

### First plasmid purification:

Here, the first plasmid is prepared by the Plasmid Mini-prep Purification kit (bought from Protech Technology Enterprise Co., Ltd.).

Firstly, 1.5 ml second bacterial solution is taken out to centrifuge at 12000 x g for 5 minutes. After the centrifugation, the bacterial precipitates are taken out.

200 µl solution I (resuspension solution), is added into the micro centrifuge tube contained with the bacterial precipitates, and then the micro centrifuge tube is oscillated until the bacteria are suspended. And 200 µl solution II (lysis solution), is added into the micro centrifuge tube and mixed with the bacterial solution contained in the micro centrifuge tube to dissolve the bacteria completely to isolate the first plasmid. Then, 200µl solution III (neutralization solution), is added into the micro centrifuge tube and mixed with the bacterial solution contained in the micro centrifuge tube. After the addition, the bacterial solution is centrifuged at 12000 x g for 5 minutes and the supernatant liquid of the bacterial solution is taken out and added to a spin column (which has DNA absorbing film), and a collection tube. Then, the spin column and the collection tube having the supernatant liquid of the bacterial solution is centrifuged at 12000 x g for 1 minute, so that the DNA in the supernatant liquid can be absorbed on the DNA absorbing film. Subsequently, 350 ml wash solution is added to the spin column and the spin column is centrifuged at 12000 x g for 1 minute, the aforementioned step is repeated again. Then, the spin column is centrifuged at 12000 x g for 3 minutes to remove the residual ethanol. Subsequently, the spin column is put into a new 1.5 ml micro centrifuge tube, and 50 µl elution buffer which is preheated to 68°C is added to the micro centrifuge tube and incubated for 5 minutes. Then, the micro centrifuge tube is centrifuged at 12000 x g for 3 minutes, and the DNA (namely, the first plasmid), in the precipitates after the centrifugation is collected; the size of the first plasmid is checked by the restriction enzyme. 10-times diluted reaction buffer and water are added into appropriate amounts of DNA and restriction enzyme (BamH I and Sal I), to form a fifth mixed solution with the volume being 15 µl. The fifth mixed solution is reacted at 37°C for at least one hour, and then added into 1% agarose gel for the electrophoresis analysis, to ensure the DNA segments of the K4 promoter to be embedded is present in the first plasmid.

### Third experiment: Cloning the human type II procollagen or collagen gene Polymerase chain reaction:

The human type II procollagen or collagen gene (hCollagen type 2 alpha 1, hCT2A1) shown as SEQ ID NO: 5 is taken as an example. Here, the sequence of SEQ ID NO: 14 is applied as the forward primer, and the sequence of SEQ ID NO: 15 is applied as the reverse primer.

Reagents according to Table 3 are added into a 0.2 ml micro test tube to proceed polymerase chain reaction to obtain the second PCR product. Here, the first stage condition of PCR is reacting at 98°C for 10 seconds; the second stage condition is reacting at 64°C for 30 seconds; the third stage condition is reacting at 72°C for 4 minutes and 30 seconds, and each stage is progressed in order for 30 cycles.

Then, the second PCR product is checked by 1% agarose gel electrophoresis, to ensure the human procollagen or collagen gene is present in the second PCR product or not.

**Table 3**

| Reagent | Usage amount |
|---|---|
| Template DNA (16ng/µl) | 10 µL |
| 5' primer (2µM) | 5 µL |
| 3' primer (2µM) | 5 µL |
| TAQ+polymerase buffer+dNTP Mixture | 25 µL |
| Sterile H₂O | 5 µL |
| Total amount | 50 µL |

Wherein, the template DNA is the cDNA of the human procollagen or collagen gene.

### Annealing reaction:

According to Table 4, reagents are added into a 0.2 ml micro test tube to proceed annealing reaction (22°C, 4 hours), to obtain the sixth mixed solution (which contains the fourth vector).

**Table 4**

| Reagent | Usage amount |
|---|---|
| Second PCR product | 6.5 µL |
| yT&A vector (third vector) | 1 µL |
| T4 DNA ligase buffer (A) | 1 µL |
| T4 DNA ligase buffer (B) | 1 µL |
| T4 DNA ligase | 0.5 µL |
| Total amount | 10 µL |

### Transformation reaction:

The sixth mixed solution is added to the prepared *E. coli* TOP 10 bacterial solution, and then the mixture is put on the ice for 30 minutes. Then, the mixture is water bathed at 42°C for 1-2 minutes to proceed the heat-shock reaction. After the heat-shock reaction, the mixture is ice bathed immediately for 3-5 minutes, and then LB medium is added to the mixture to total volume of 1L, to obtain a third bacterial solution. The third bacterial solution is undergone agitation culture at 37°C for 1 hour. 100 µl of 100 mM IPTG (isopropyl-b-d-thiogalactopyranoside) and 20 µl of 50 mg/ml X-gel (5-bromo-4-chloro-3-indolyl-beta-D-galactopyranoside) are coated on the LB/Amp medium, and the third bacterial solution is uniformly coated in the LB/Amp medium to incubate at 37°C for 12-16 hours.

Here, the quick screening, such as blue white screen, etc., can be applied to roughly isolate the strains having the human procollagen or collagen gene. As the procedure of the quick screening is well known by those skilled in the art, additional details relating to the procedure of the quick screening are not provided.

### Second plasmid purification

A white single colony is picked up by micropipettes and is mixed with 10 ml LB/amp medium in the test tube to incubate for 8 hours. Then, several colonies are chosen and undergone agitation culture at 37°C for 14 hours, to be prepared for purifying the second plasmid having the DNA segments of the human type II procollagen or collagen gene (referred to hCT2Al-yt&A). Here, the second plasmid is prepared by the Plasmid Mini-prep Purification kit (bought from Protech Technology Enterprise Co., Ltd.), and the size of the second plasmid is checked by the restriction enzyme (BamH and Sal I). Subsequently, 1% agarose gel is applied in the electrophoresis analysis, to ensure the DNA segments of the human type II procollagen or collagen gene to be embedded is present in the second plasmid. As the purification procedure is well known by those skilled in the art, additional details relating to the purification procedure are not provided.

### Fourth embodiment: Constructing the upstream gene applicable for the human type II procollagen or collagen gene

According to Table 5, restriction enzymes (ApaI and BamHI), is applied to cut the first plasmid (K4-yt&A) and the first expression vector (pT2-LFABP-tTa) (reacting at 37°C for at least 2 hours), to obtain a seventh mixed solution (which contains K4), and an eighth mixed solution (which contains pT2-tTa), respectively.

**Table 5**

| Reagent | Usage amount |
|---|---|
| K4-yt&A or pT2-LF-tTa | 16 µl |
| *Apa*1 | 1 µl |
| *BamH 1* | 1 µl |
| T4 DNA ligase Buffer A | 2 µl |
| Total amount | 20 µl |

After the reaction, the seventh mixed solution (which contains K4), and the eighth mixed solution (which contains pT2-tTa), are injected into 1% agarose gel to undergo electrophoresis analysis respectively, and DNA segments with certain sizes are cut from the seventh mixed solution and the eighth mixed solution, respectively. Then, DNA products (that is, K4 and pT2-tTa), are recycled by the Gel Elution Kit (bought from GeneMark).

According to Table 6, the two DNA products (K4 and pT2-tTa), are mixed at 70°C for 3 minutes, and then the mixture is ice bathed immediately. Subsequently, other reagents are added into the mixture uniformly; and the mixture is incubated at 22°C for 4 hours to undergo the annealing reaction, to obtain a ninth mixed solution (which contains the upstream gene).

**Table 6**

| Reagent | Usage amount |
|---|---|
| K4 | 7.5 µl |
| pT2-tTa | 1.5 µl |
| 2 ligase buffer | 10 µl |
| T4 DNA ligase | 1 µl |
| Total amount | 20 µl |

The E. coli TOP 10 strains are applied as the competent cells, and the upstream gene in the ninth mixed solution is transformed to the E. coli TOP 10 to incubate (strain culture). Subsequently, strains having the upstream gene are selected from the cultivated strains and purified by the purified kit to obtain the upstream gene (pT2-K4-tTa).

As the procedures of the strain culture, the strain isolation and the purification are well known by those skilled in the art, additional details relating to these procedures are not provided.

### Fifth experiment: Constructing the recombinant plasmid

According to Table 7, restriction enzymes (Sal I and BamH I), are applied to cut pT2-K4-tTa and hCT2A1-yt&A (at 37°C for at least two hours), respectively to obtain a tenth mixed solution (which contains pT2-K4), and an eleventh mixed solution (which contains hCT2A1).

**Table 7**

| Reagent | Usage amount |
|---|---|
| pT2-K4-tTa or hCT2A1-yt&A | 16 µl |
| Sal I | 1 µl |
| BamH I | 1 µl |
| Restriction Enzyme Buffer | 2 µl |
| Total amount | 20 µl |

After the reaction, the tenth mixed solution (which contains pT2-K4), and the eleventh mixed solution (which contains hCT2A1), are injected into 1% agarose gel to undergo electrophoresis analysis respectively, and DNA segments with certain sizes are cut from the tenth mixed solution and the eleventh mixed solution, respectively. Then, DNA products (that is, pT2-K4 and hCT2A1), are recycled by the Gel Elution Kit (DNA recycle kit, bought from GeneMark).

According to Table 8, the two DNA products (pT2-K4 and hCT2A1), are mixed at 70°C for 3 minutes, and then the mixture is ice bathed immediately. Subsequently, other reagents are added into the mixture uniformly; and the mixture is incubated at 22°C for 4 hours to undergo the annealing reaction to obtain a twelfth mixed solution (which contains the second expression vector).

**Table 8**

| Reagent | Usage amount |
|---|---|
| hCT2A1 | 7.5 µl |
| pT2-K4 | 1.5 µl |
| 2 ligase buffer | 10 µl |
| T4 DNA ligase | 1 µl |
| Total amount | 20 µl |

The *E*. *coli* TOP 10 strains are applied as the competent cells, and the second expression vector in the twelfth mixed solution is transformed to the E. coli TOP 10 to incubate (strain culture). Subsequently, strains having the second expression vector are selected from the cultivated strains and purified by the purified kit to obtain the second expression vector (pT2-K4-hCT2A1).

Here, the green fluorescent protein gene is used as the report gene.

According to Table 9, restriction enzymes (Sal I and Cla I), are applied to cut the report gene (IRES-hrGFP), and pT2-K4-hCT2A1 (at 37°C for at least two hours), respectively to obtain a thirteenth mixed solution (which contains IRES-hrGFP), and a fourteenth mixed solution (which contains pT2-K4-hCT2A1).

**Table 9**

| Reagent | Usage amount |
|---|---|
| IRES-hrGFP or pT2-K4-hCT2A1 | 16 µl |
| Sal I | 1 µl |
| Cla I | 1 µl |
| Restriction Enzyme Buffer | 2 µl |
| Total amount | 20 µl |

After the reaction, the thirteenth mixed solution (which contains IRES-hrGFP), and the fourteenth mixed solution (which contains pT2-K4-hCT2A1), are injected into 1% agarose gel to undergo electrophoresis analysis respectively, and DNA segments with certain sizes are cut from the thirteenth mixed solution and the fourteenth mixed solution, respectively. Then, DNA products (that is, IRES-hRGEP and pT2-K4-hCT2A1), are recycled by the Gel Elution Kit (DNA recycle kit, bought from GeneMark).

According to Table 10, the two DNA products (IRES-hRGEP and pT2-K4-hCT2A1), are mixed at 70°C for 3 minutes, and then the mixture is ice bathed immediately. Subsequently, other reagents are added into the mixture uniformly; and the mixture is incubated at 22°C for 4 hours to undergo the annealing reaction to obtain a fifteenth mixed solution (which contains the recombinant plasmid).

**Table 10**

| Reagent | Usage amount |
|---|---|
| IRES-hrGFP | 7.5 µl |
| pT2-K4-hCT2A1 | 1.5 µl |
| 2 ligase buffer | 10 µl |
| T4 DNA ligase | 1 µl |
| Total amount | 20 µl |

The *E*. *coli* TOP 10 strains are applied as the competent cells, and the recombinant plasmid in the fifteenth mixed solution is transformed to the *E*. *coli* TOP 10 to incubate (strain culture). Subsequently, strains having the recombinant plasmid are selected from the cultivated strains. As the procedures of the strain culture and the strain isolation are well known by those skilled in the art, additional details relating to these procedures are not provided.

Herein, after the recombinant plasmid is transformed to the *E*. *coli* TOP 10, the cryopreservation medium (20% Glycerol: LB medium=1:1) can be added.

### Recombinant plasmid purification:

The strains which are selected and sequencing ensured are inoculated into 200 ml LB/Amp medium to undergo agitation culture at 37°C with 175 rpm. After the agitation culture, the mixture is centrifuged at 13000 x g for 5 minutes to obtain the bacterial precipitates.

10 ml equilibration buffer is added into the balance column in advance. 4ml cell suspension buffer is added into the centrifuged bacterial precipitates to oscillate until no precipitates exists (that is, all the bacterial precipitates is dissolved), to obtain a sixteenth mixed solution.

4 ml cell lysis solution is added and carefully mixed with the sixteenth mixed solution; then the mixture is incubated in room temperature for 5 minutes. Next, 4 ml neutralization buffer solution is added and carefully mixed with the mixture and carefully mixed with the mixture, and then the mixture is centrifuged at 15000 x g for 10 minutes (25°C). After the centrifugation, the supernatant liquid of the mixture is absorbed and transferred to the aforementioned balance column and washed by using 10 ml wash solution for two times, and then 5 ml elution buffer is added to the balance column and the DNA solution eluted out is collected.

Subsequently, 3.5 ml isopropanol is added to the collected DNA solution and the mixture is centrifuged at 15000 x g for 30 minutes. After the centrifugation, the supernatant liquid is poured out and 3 ml 70% alcohol is added to the precipitates left. Then, the mixture is centrifuged at 15000 x g for 5 minutes so as to remove the alcohol. After the alcohol is removed, 200 µl TE buffer is added to the air-dried DNA to dissolve the DNA, then the solution which contains the recombinant plasmid (pT2-K4-hCT2A1-IRES-hrFGP) is obtained. After the DNA is dissolved completely, 2 µl solution is taken out to measure the optical density at 260 nm (OD 260 nm), so as to quantify the DNA, and the rest solution is stored at -80°C.

The cooling procedure includes sequentially storing it at 4°C for 1 hour, at -20°C for 1 hour, and at -80°C for 1 hour.

### Sixth experiment: Cloning the human type I procollagen or collagen gene

The human type I procollagen or collagen gene (hCollagen type 1 alpha 1, hCT1A1), shown as SEQ ID NO: 3 is taken as an example. Here, the sequence of SEQ ID NO: 8 is applied as the forward primer, and the sequence of SEQ ID NO: 9 is applied as the reverse primer.

As the procedures of cloning the human type I procollagen or collagen gene is substantially similar to the procedures of cloning the human type II procollagen or collagen gene as described in the third experiment, additional details relating to these procedures are not provided. In this experiment, the second plasmid having the DNA segments of the human type I procollagen or collagen gene is obtained, referred to as hCT1A1-yt&A.

### Seventh experiment: Constructing the upstream gene applicable for the human type I procollagen or collagen gene

According to Table 11, restriction enzymes (Apa I and BamH I), are applied to cut the first plasmid (K4-yt&A), and the first expression vector(pT2-LF2.9-AmCyan1) (at 37°C for at least two hours), respectively to obtain a seventeenth mixed solution (which contains K4), and an eighteenth mixed solution (which contains pT2-AmCyan1).

**Table 11**

| Reagent | Usage amount |
|---|---|
| K4-yt&A or pT2-LF2.9-AmCyan1 | 16 µl |
| Apa I | 1 µl |
| BamH I | 1 µl |
| Buffer A | 2 µl |
| Total amount | 20 µl |

After the reaction, the seventeenth mixed solution (which contains K4), and the eighteenth mixed solution (which contains pT2-AmCyan1), are injected into 1% agarose gel to undergo electrophoresis analysis respectively, and DNA segments with certain sizes are cut from the seventeenth mixed solution and the eighteenth mixed solution, respectively. Then, DNA products (that is, K4 and pT2-AmCyan1), are recycled by the Gel Elution Kit (DNA recycle kit, bought from GeneMark).

According to Table 12, the two DNA products (K4 and pT2-AmCyan1) are mixed at 70°C for 3 minutes, and then the mixture is ice bathed immediately. Subsequently, other reagents are added into the mixture uniformly; and the mixture is incubated at 22°C for 4 hours to undergo the annealing reaction to obtain a nineteenth mixed solution (which contains the second expression vector).

**Table 12**

| Reagent | Usage amount |
|---|---|
| K4 | 7.5 µl |
| pT2-AmCyan1 | 1.5 µl |
| 2 ligase buffer | 10 µl |
| T4 DNA ligase | 1 µl |
| Total amount | 20 µl |

The *E*. *coli* TOP 10 strains are applied as the competent cells, and the second expression vector in the nineteenth mixed solution is transformed to the *E*. *coli* TOP 10 to incubate (strain culture). Subsequently, strains having the upstream gene are selected from the cultivated strains and purified by the purified kit to obtain the upstream gene (pT2-K4-AmCyan1).

As the procedures of the strain culture, the strain isolation and the purification are well known by those skilled in the art, additional details relating to these procedures are not provided.

### Eighth experiment: Constructing the recombinant plasmid

According to Table 13, restriction enzymes (Sal I and Age I), are applied to cut pT2-K4-AmCyan1 and hCT1A1-yt&A (at 37°C for at least two hours), respectively to obtain a twentieth mixed solution (which contains pT2-K4), and a twenty-first mixed solution (which contains hCT1A1).

**Table 13**

| Reagent | Usage amount |
|---|---|
| pT2-K4-AmCyan1 or hCT1A1-yt&A | 16 µl |
| Sal I | 1 µl |
| Age I | 1 µl |
| Buffer D | 2 µl |
| Total amount | 20 µl |

After the reaction, the twentieth mixed solution (which contains pT2-K4), and the twenty-first mixed solution (which contains hCT1A1), are injected into 1% agarose gel to undergo electrophoresis analysis respectively, and DNA segments with certain sizes are cut from the twentieth mixed solution and the twenty-first mixed solution, respectively. Then, DNA products (that is, pT2-K4 and hCT1A1), are recycled by the Gel Elution Kit (DNA recycle kit, bought from GeneMark).

According to Table 14, the two DNA products (that is, pT2-K4 and hCT1A1), are mixed at 70°C for 3 minutes, and then the mixture is ice bathed immediately. Subsequently, other reagents are added into the mixture uniformly; and the mixture is incubated at 22°C for 4 hours to undergo the annealing reaction to obtain a twenty-second mixed solution (which contains the recombinant plasmid).

**Table 14**

| Reagent | Usage amount |
|---|---|
| hCT1A1 | 7.5 µl |
| pT2-K4 | 1.5 µl |
| 2 ligase buffer | 10 µl |
| T4 DNA ligase | 1 µl |
| Total amount | 20 µl |

The recombinant plasmid in the twenty-second mixed solution is transformed to the *E*. *coli* TOP 10 to incubate (strain culture). Subsequently, strains having the recombinant plasmid are selected from the cultivated strains and purified by the purified kit to obtain the recombinant plasmid (referred to as pT2-K4-hCT1A1 - deposited as DSM 27616).

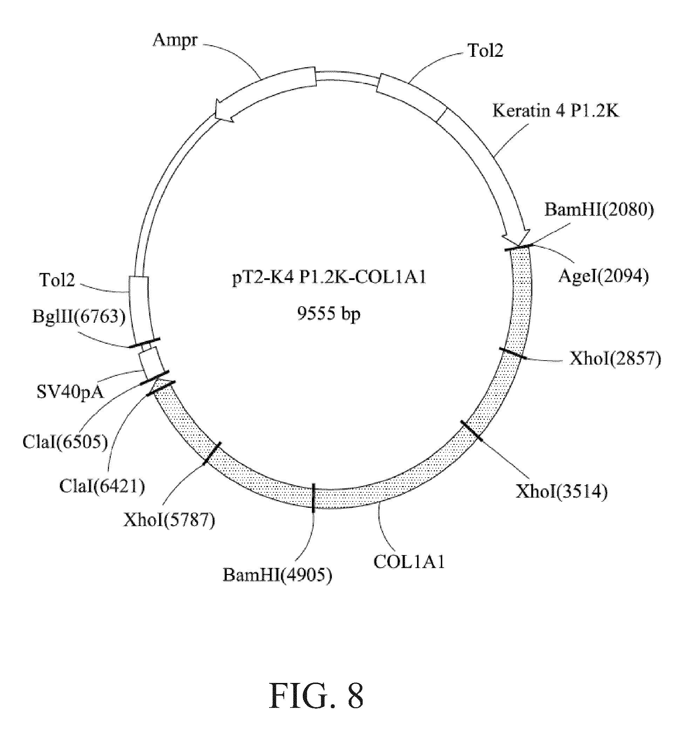

Then, the recombinant plasmid of pT2-K4-hCT1A1 (deposited with accession number: DSM 27616) is transformed to the *E*. *coli* TOP 10, to incubate (strain culture). Subsequently, strains having the recombinant plasmid of pT2-K4-hCT1A1 (deposited with accession number: DSM 27616), as shown in FIG. 8, are selected from the cultivated strains and purified by the purified kit. In this experiment, the procedures are substantially similar as that described in the fifth experiment.

In FIG. 8, K4 P1.2K (keratin 4 P1.2K) refers to K4 1.2kb promoter of the Zebra fish, COL1A1 refers to cDNA of the human type I, alpha 1 collagen, and Tol2 refers to Tol2 transposase recognition sequence for transposition. A cutting site of LF2.9 promoter ApaI/BamHI of pT2-LF2.9-AmCyan1 is displaced by the K4 promoter ApaI/BamHI of the Zebra fish, to form pT2-K4-AmCyan1. A cutting site of Amcyan1 AgeI/XhoI of the pT2-K4-AmCyan1 is displaced by hCT1A1 AgeI/SalI, to obtain the pT2-K4-hCT1A1 (deposited with accession number: DSM 27616). Herein, cutting sites of SalI and XhoI are isoschizomers, and both SalI and XhoI are destroyed after ligation.

### Ninth experiment: Preparation of the fish embryos:

Wild type grown-up zebrafishes (*Danio rerio*, AB strain), are raised at 28°C, the condition of the photoperiod is set as 14 hours daylight/ 10 hours darkroom, with raising feeding stuffs at the morning and the evening.

The male zebrafishes and the female zebrafishes are mixed cultured at the morning regularly (male: female = 2:1), to copulate with each other to lay eggs. After the copulation is finished for about 30 to 40 minutes, the embryos are collected (at this time, the embryos are at the 1-cell stage), to be applied in gene transfection.

### Tenth experiment: Preparation of the recombinant plasmid to be introduced:

According to Table 15, reagents are mixed uniformly and incubated at 37°C to react with each other for at least 3 hours, to convert the constructed recombinant plasmid (as the product of the fifth embodiment or the eighth embodiment) into linear DNA by using proper restriction enzymes.

**Table 15**

| Reagent | Usage amount |
|---|---|
| Recombinant plasmid | Appropriate amount |
| Restriction enzyme | 2 µl |
| BSA (10mg/ml) | 1 µl |
| Sterile water is added until total volume is | 20 µl |

Gel Elution Kit (bought from GeneMark), is applied to recycle the DNA products, and appropriate amount of sterile water is added to re-dissolve DNA to make the final concentration of the linear DNA is 0.1 µg/µl.

Before the microinjection, 40 µl linear DNA is uniformly mixed with 5 µl tracing dye (here, phenol red is applied as the tracing dye), to obtain a twenty-third mixed solution. The twenty-third mixed solution is stored at -20°C for the microinjection.

### Eleventh experiment: Introducing the recombinant plasmid

The collected embryos are disposed in the groove of the agarose gel, so that the embryos are incubated on the agarose gel. Then, the aforementioned twenty-third mixed solution is injected into the incubated embryos via microinjection.

After the microinjection, the embryos are moved to the incubation tank and cultivated for 24 hours. Then, the green fluorescent protein (hrGFP), expressed on the embryos are observed by the fluorescent microscope and are photographed for record. Here, the expression of the green fluorescent protein means the expression of the human procollagen or collagen gene.

The transgenic embryos are grown as the parent-generation (F0 generation) transgenic zebrafishes. Then, the F0 generation transgenic zebrafishes are grown to be sexually matured (about 3 to 5 months), and copulated with wild type stock zebrafishes to get the F1 generation (child generation), transgenic zebrafishes which express fluorescence. Then, after the F1 generation transgenic zebrafishes are raised for 3 to 5 months, the F1 generation transgenic zebrafishes can copulate to produce the F2 generation (child generation) transgenic zebrafishes.

Here, K4 can drive the human procollagen or collagen gene to express on the fish scales; that is, to make the transgenic zebrafishes secret the human procollagen or collagen gene on the fish scales.

Here, the report gene is applied to ensure the expression of the introduced recombinant gene structure, but embodiments of the disclosure are not limited thereto. For those who are skilled in the art knows that the fishes having the recombinant gene structures can be checked by many proper methods. For example, the chromosomes of the fishes are checked by PCR, to detect whether the fishes have the recombinant gene structures or not. Or, the expression product (the human procollagen or collagen), of the recombinant gene structure can be detected so that the fishes which express the transgenic genes practically can be detected. In addition, related checking method applicable for the transgenic animals can also be applied for the identification of the transgenic fishes, for instance, northern blot or southern blot methods are applied to detect whether the fishes have the nucleic acids of transgenic genes. Or, PCR and other nucleic acid amplification techniques with sequence specificity can be applied.

### Twelfth experiment: Introducing the recombinant plasmid

Further, as referring to the gene transfection techniques developed by the Japanese scholar Kawakami, Tol2 transponase mRNA are synthesized by in-vitro transcription and injected into the embryos of the zebra fishes along with the twenty-third mixed solution. The gene transfection techniques can be refer to the publication "Transposon tools and methods in zebrafish Dev. Dyn. 234(2): 224-254, 2005".

### Thirteenth experiment: Preparation of the biomaterial

One or many fish scales are collected from the transgenic fish, and the collected fish scales are undergone the washing procedure, the cell removing procedure, the dehydration procedure and other procedures, so that the collected fish scale(s) is/are treated to be a biomaterial having the human procollagen or collagen.

Here, an extrusion procedure can be further undergone to extrude the aforementioned fish scale(s) to be certain shaped and/or certain sized biomaterials having the human procollagens or collagens.

Further, before the extrusion procedure, a grinding procedure can be applied to grind the fish scale(s). In addition, after the grinding procedure, the fish scale particles can be filtered to be separated by different sizes, so that the biomaterials having the human procollagens or collagens can be provided in required sizes.

Furthermore, the aforementioned biomaterials can be sterilized, so that the sterilize biomaterials can be prepared.

The procedures related to the preparation of the aforementioned biomaterials can be referred to US Patent Application No. 12/324,551, US Patent Application No. 12/659,282, US Patent Application No. 12/880,349 and US Patent Application No. 13/019,134.

### Fourteenth experiment: Preparation of the E. coli TOP 10 strains

Here, the *E*. *coli* TOP 10 strains are used as the competent cells, and the genotype thereof is F⁻mcrA Δ(mrr-hsdRMS-mcrBC) ϕ80lacZΔM15 ΔlacX74 recA1 araD139 Δ(ara-leu) 7697 galU galK rpsL (StrR) endA1 nup^{Gλ-}.

*E*. coli TOP 10 is cultured in LB medium overnight, and then the bacterial solution is added into 5 ml LB/Amp medium (LB/Amp medium: bacterial solution=100:1) to incubate at 37°C with vigorous shaking in an incubator until OD600 of 0.5-0.6.

The following steps are operated on ice. The *E*. *coli* TOP 10 bacterial solution is stayed on ice for 10 minutes, and the 2 ml of the bacterial solution having O.D600 of 0.5-0.6 is centrifuged at 4000 x g, 4°C for 3 minutes or at 5000 x g, 4°C for 4 minutes. After the centrifugation, the supernatant liquid is removed by a pippet, and then is mix uniformly with 1 ml 200 mM (or 250mM) CaCl₂, so as to completely re-dissolved the bacteria. The re-dissolved solution is put on the ice for 10 minutes, and then centrifuged at 5000 x g, 4°C for 4 minutes. After the centrifugation, the supernatant liquid is removed and then added with 150µl-200ml CaCl₂ to re-dissolve completely. Before transformation, the re-dissolved bacterial solution is stayed on ice for over 10 minutes.

Based on the above, the recombinant vector, the transgenic fish using the same and the biomaterial using the same according to the invention can be applied to provide a transgenic fish that secrets the human procollagens or collagens from part(s) of the transgenic fish, so that part(s) of the transgenic fish that secrets the human procollagens or collagens can be treated as the biomaterials (e.g. the biomaterial having the human procollagens or collagens and the inorganic substances) and/or can be treated to purify the human procollagens or collagens.

While the disclosure has been described by the way of example and in terms of the preferred embodiments, it is to be understood that the invention need not be limited to the disclosed embodiments. On the contrary, it is intended to cover various modifications and similar arrangements included within the spirit and scope of the appended claims, the scope of which should be accorded the broadest interpretation to encompass all such modifications and similar structures.

## Claims

1. A biomaterial comprising a recombinant human procollagen or collagen obtained from and secreted by a fish.

2. The biomaterial of claim 1 further comprising at least one fish scale of the fish, each having the recombinant human procollagen or collagen

3. The biomaterial of claim 1 wherein the recombinant human procollagen or collagen is expressed by at least one human procollagen or collagen gene (hCG) in the fish.

4. A transgenic fish egg or embryo, comprising:
a fish chromosome; and
a recombinant vector, inserted in the fish chromosome, the recombinant vector having a human procollagen or collagen gene (hCG).

5. The recombinant fish egg or embryo of claim 4, wherein the recombinant vector further comprises an upstream gene (UG), enabling expression of the human procollagen or collagen gene (hCG), and the human procollagen or collagen gene (hCG) is connected to downstream of the upstream gene (UG).

6. The transgenic fish egg or embryo of claim 5, wherein the upstream gene (UG) is a promoter existed in a fish, an enhancer existed in a fish, or combination thereof.

7. The transgenic fish egg or embryo of claim 5, wherein the recombinant vector further comprises a report gene (RG), connected to downstream of the human procollagen or collagen gene (hCG).

8. The transgenic fish egg or embryo of claim 4, wherein the recombinant vector further comprises a report gene (RG), connected to downstream of the human procollagen or collagen gene (hCG).

9. A recombinant vector comprising:
an upstream gene (UG), constructed from a fish; and
a human procollagen or collagen gene (hCG), connected to downstream of the upstream gene (UG).

10. The recombinant vector of claim 9, further comprising a report gene (RG), connected to downstream of the human procollagen or collagen gene (hCG).

11. The recombinant vector of claim 9, wherein the upstream gene (UG) is a promoter existed in the fish, an enhancer existed in a fish, or combination thereof.


SEQUENCE LISTING

<110>  Body Organ Biomedical Corp.

<120>  RECOMBINANT VECTOR, TRANSGENIC FISH EGG USING THE SAME AND
       BIOMATERIAL USING THE SAME

<130>  P 53825

<140>
<141>  2013-08-29

<150>  US 61/694,877
<151>  2012-08-30

<160>  17

<170>  PatentIn version 3.5

<210>  1
<211>  1170
<212>  DNA
<213>  Danio rerio, AB strain

<400>  1
ggttcgacat tggattttgg tcgctttcca acacaaccta aatccaccaa atattaactt      60

cctatgacat cgttattgga cgtcaaaata acaatatcct tagatgctgg ctagactttg     120

aatttaggtc accacaacct atatttaacc taatattaac atcttatgat gttgtgtgcc     180

tgctgggcaa taactaaatg cactacagaa tgttacgttt acacacatgt aaattacatg     240

taaatgcatc agcttttcac agcataatac tcactactta ctactcttga gtacttttaa     300

aaaagctact tttcactcat actttgagta atatttacaa ctgatacttt tactcgcact     360

acatttttag gcatgtattg atatttttac tatgattttt cagtactctt tccactactg     420

cagccctccc catacataat cgtatgttta cacatatggt ggagtttaga gccataaact     480

acattagctt tgttagccgc tagcattact gtgcagaatt gtgtgtgtgc acattttcca     540

atatcaatac agaaggaaac tgtgttccct gttcccttgt aaatctcaac aatgcaactg     600

ttcagctcag ggggaaaaat gccctgccag atccaaacgc tggcaaaagt gaatggaaaa     660

aagcctttca ttaatgtgaa agttgctgcg cgccccaccc agataaaaag agcagaggtt     720

aacatgctct ctacggctgt ccagccaacc agatactgag gcagaaacac acccgctggc     780

agatggtgag agctacactg tctttttcag agtttctact ggaatgcctg tcctcaagtc     840

tcaagcctct ccttgcattt tctcattcca cctggggcaa agccccaggc tgggtgtgac     900

aacatttatc ttaccacttt ctctctgtac ctgtctaaca ggtagggtgt gtgtgagagt     960

gcgtatgtgt gcaagtgtgt gtgtgtgtga gagcagtcag ctccaccctc tcaagagtgt    1020

gtataaaatt ggtcagccag ctgctgagag acacgcagag ggactttgac tctcctttgt    1080

gagcaacctc ctccactcac tcctctctca gagagcactc tcgtacctcc ttctcagcaa    1140

ctcaaagaca caggcatcat gtcaaccagg 1170

<210> 2
<211> 1570
<212> DNA
<213> Danio rerio, AB strain

<400> 2
gtcctgtttt ctagatcaca ggtatttcaa agcatcattt tcatcacaac aattatgcgt 60

aattgttttt ttaccattct cagatttcaa atcctgtgac attaaagctg ggttcacaat 120

tttttttaac gtcatttata attgttccct gtcagacgtt ggacatgatc ccagtgagat 180

cttgggtaaa agccatgaag cagtgtgtga catcaataat attttatgtc agacagtatc 240

atcttgtcac acccaagact acagttccct ttatggattt tttcaaaatt tgagcaaaat 300

tgttaccaaa agcaacagca cagtagctca gtggttagca ctgttacgtc acagcaagaa 360

tgtcgctggt ttgagtcccg actgtgctgg tgtgggtttc ctccgggtgg tccggtttcc 420

cccacagtcc aaatacatgc gctataggtg aattaggtaa actgaattgg ctgtagtgta 480

tgtgtatgtg tgtgaatgca agcatgtatg ggtgtttccc aatactaggt tggcaactgg 540

aagaccatcc gctgtgtgaa acatacgcca gaatagttgg cggttcattc cgctgtgaca 600

acgttaaata agagactaag ctgaagaaaa atgaacgaat gaatgctagc aaaatcatac 660

catgtaaatc tgggaagaac atggtcaagc tattcctcag ttactgaact tcaaaccatt 720

tgcttttatc accttgtttt tctatcttat caacttgttt ttctacaact tatcaatgtt 780

ttcaaacaca acatatttgg gcagttatgc aattttgagc aggcccttaa aaggtaaaaa 840

gcaagcgagc ctatttgtag ggctgcagtt tgcagaaaca tctcaaagaa taaacacaga 900

gcttttagat attttaaaga gatgctcttc aatcatgttg ttgcaaactt cttcgtaagc 960

ttcttcaggg gagttttggg tgaagtcccc ccttaagaat ctgagttttg taaacatgtt 1020

tacatttaat ccacatgtgt ccactctttt tggaaaatgc acatttaggt gtaacctgtc 1080

ccaaaggtgc ctgattgaaa tcataaacca ctgtttactt tctcaaaccc tgttcttctc 1140

agctaaacat gctatttaag ttttcactga agcattgtgg gactgaaagc acaaaatgag 1200

gctgtatctt gttgcagtat gtatgtgtct agatgagctt ggggattggg ttcagatgga 1260

gggagtcaga ggggggagat cgaggcagga aatgagaaag agagagagga tgtgggacgg 1320

gcgttagaga acactgaaaa aagtaatgca aaaggaaaaa aaccggacca tgtgggcagg 1380

gtcttagttg gtcagtgcgc gtgtgtgtgt ttgagtcaaa aagagaaaag gaggaacaga 1440

aagagtttgg tgggggtata aaagctgtac agttctcttc tagtggctct caagggttga 1500

catccctcag cgcacactca tcttctactg cagccagagc ttcattcctc caaaaccatg 1560

agcttctacg 1570

<210> 3
<211> 4752
<212> DNA
<213> Homo sapiens collagen, type I, alpha 1 (hCT1A1)

<400> 3
agacgggagt ttctcctcgg ggtcggagca ggaggcacgc ggagtgtgag gccacgcatg 60

agcggacgct aaccccctcc ccagccacaa agagtctaca tgtctagggt ctagacatgt 120

tcagctttgt ggacctccgg ctcctgctcc tcttagcggc caccgccctc ctgacgcacg 180

gccaagagga aggccaagtc gagggccaag acgaagacat cccaccaatc acctgcgtac 240

agaacggcct caggtaccat gaccgagacg tgtggaaacc cgagccctgc cggatctgcg 300

tctgcgacaa cggcaaggtg ttgtgcgatg acgtgatctg tgacgagacc aagaactgcc 360

ccggcgccga agtccccgag ggcgagtgct gtcccgtctg ccccgacggc tcagagtcac 420

ccaccgacca agaaaccacc ggcgtcgagg gacccaaggg agacactggc ccccgaggcc 480

caaggggacc cgcaggcccc cctggccgag atggcatccc tggacagcct ggacttcccg 540

gaccccccgg accccccgga cctcccggac cccctggcct cggaggaaac tttgctcccc 600

agctgtctta tggctatgat gagaaatcaa ccggaggaat ttccgtgcct ggccccatgg 660

gtccctctgg tcctcgtggt ctccctggcc cccctggtgc acctggtccc caaggcttcc 720

aaggtccccc tggtgagcct ggcgagcctg gagcttcagg tcccatgggt ccccgaggtc 780

ccccaggtcc ccctggaaag aatggagatg atggggaagc tggaaaacct ggtcgtcctg 840

gtgagcgtgg gcctcctggg cctcagggtg ctcgaggatt gcccggaaca gctggcctcc 900

ctggaatgaa gggacacaga ggtttcagtg gtttggatgg tgccaaggga gatgctggtc 960

ctgctggtcc taagggtgag cctggcagcc ctggtgaaaa tggagctcct ggtcagatgg 1020

gcccccgtgg cctgcctggt gagagaggtc gccctggagc ccctggccct gctggtgctc 1080

gtggaaatga tggtgctact ggtgctgccg ggccccctgg tcccaccggc cccgctggtc 1140

ctcctggctt ccctggtgct gttggtgcta agggtgaagc tggtccccaa gggccccgag 1200

gctctgaagg tccccagggt gtgcgtggtg agcctggccc ccctggccct gctggtgctg 1260

ctggccctgc tggaaaccct ggtgctgatg gacagcctgg tgctaaaggt gccaatggtg 1320

ctcctggtat tgctggtgct cctggcttcc ctggtgcccg aggcccctct ggaccccagg 1380

gccccggcgg ccctcctggt cccaagggta acagcggtga acctggtgct cctggcagca 1440

aaggagacac tggtgctaag ggagagcctg gccctgttgg tgttcaagga ccccctggcc 1500

ctgctggaga ggaaggaaag cgaggagctc gaggtgaacc cggacccact ggcctgcccg 1560

gaccccctgg cgagcgtggt ggacctggta gccgtggttt ccctggcgca gatggtgttg 1620

ctggtcccaa gggtcccgct ggtgaacgtg gttctcctgg ccctgctggc cccaaaggat 1680

ctcctggtga agctggtcgt cccggtgaag ctggtctgcc tggtgccaag ggtctgactg 1740

gaagccctgg cagccctggt cctgatggca aaactggccc ccctggtccc gccggtcaag 1800

atggtcgccc cggaccccca ggcccacctg gtgcccgtgg tcaggctggt gtgatgggat 1860

tccctggacc taaaggtgct gctggagagc ccggcaaggc tggagagcga ggtgttcccg 1920

gaccccctgg cgctgtcggt cctgctggca aagatggaga ggctggagct cagggacccc 1980

ctggccctgc tggtcccgct ggcgagagag gtgaacaagg ccctgctggc tcccccggat 2040

tccagggtct ccctggtcct gctggtcctc caggtgaagc aggcaaacct ggtgaacagg 2100

gtgttcctgg agaccttggc gcccctggcc cctctggagc aagaggcgag agaggtttcc 2160

ctggcgagcg tggtgtgcaa ggtccccctg gtcctgctgg tccccgaggg gccaacggtg 2220

ctcccggcaa cgatggtgct aagggtgatg ctggtgcccc tggagctccc ggtagccagg 2280

gcgcccctgg ccttcaggga atgcctggtg aacgtggtgc agctggtctt ccagggccta 2340

agggtgacag aggtgatgct ggtcccaaag gtgctgatgg ctctcctggc aaagatggcg 2400

tccgtggtct gaccggcccc attggtcctc ctggccctgc tggtgcccct ggtgacaagg 2460

gtgaaagtgg tcccagcggc cctgctggtc ccactggagc tcgtggtgcc cccggagacc 2520

gtggtgagcc tggtcccccc ggccctgctg gctttgctgg cccccctggt gctgacggcc 2580

aacctggtgc taaaggcgaa cctggtgatg ctggtgctaa aggcgatgct ggtccccctg 2640

gccctgccgg acccgctgga ccccctggcc ccattggtaa tgttggtgct cctggagcca 2700

aaggtgctcg cggcagcgct ggtccccctg gtgctactgg tttccctggt gctgctggcc 2760

gagtcggtcc tcctggcccc tctggaaatg ctggaccccc tggccctcct ggtcctgctg 2820

gcaaagaagg cggcaaaggt ccccgtggtg agactggccc tgctggacgt cctggtgaag 2880

ttggtccccc tggtccccct ggccctgctg gcgagaaagg atcccctggt gctgatggtc 2940

ctgctggtgc tcctggtact cccgggcctc aaggtattgc tggacagcgt ggtgtggtcg 3000

gcctgcctgg tcagagagga gagagaggct tccctggtct tcctggcccc tctggtgaac 3060

ctggcaaaca aggtccctct ggagcaagtg gtgaacgtgg tccccctggt cccatgggcc 3120

cccctggatt ggctggaccc cctggtgaat ctggacgtga gggggctcct ggtgccgaag 3180

gttcccctgg acgagacggt tctcctggcg ccaagggtga ccgtggtgag accggccccg 3240

ctggaccccc tggtgctcct ggtgctcctg gtgcccctgg ccccgttggc cctgctggca 3300

agagtggtga tcgtggtgag actggtcctg ctggtcccgc cggtcctgtc ggccctgttg 3360

gcgcccgtgg ccccgccgga ccccaaggcc cacgtggtga caagggtgag acaggcgaac 3420

agggcgacag aggcataaag ggtcaccgtg gcttctctgg cctccagggt ccccctggcc 3480

ctcctggctc tcctggtgaa caaggtccct ctggagcctc tggtcctgct ggtccccgag 3540

gtccccctgg ctctgctggt gctcctggca aagatggact caacggtctc cctggcccca 3600

ttgggccccc tggtcctcgc ggtcgcactg gtgatgctgg tcctgttggt ccccccggcc 3660

ctcctggacc tcctggtccc cctggtcctc ccagcgctgg tttcgacttc agcttcctgc 3720

cccagccacc tcaagagaag gctcacgatg gtggccgcta ctaccgggct gatgatgcca 3780

atgtggttcg tgaccgtgac ctcgaggtgg acaccaccct caagagcctg agccagcaga 3840

tcgagaacat ccggagccca gagggcagcc gcaagaaccc cgcccgcacc tgccgtgacc 3900

tcaagatgtg ccactctgac tggaagagtg gagagtactg gattgacccc aaccaaggct 3960

gcaacctgga tgccatcaaa gtcttctgca acatggagac tggtgagacc tgcgtgtacc 4020

ccactcagcc cagtgtggcc cagaagaact ggtacatcag caagaacccc aaggacaaga 4080

ggcatgtctg gttcggcgag agcatgaccg atggattcca gttcgagtat ggcggccagg 4140

gctccgaccc tgccgatgtg gccatccagc tgaccttcct gcgcctgatg tccaccgagg 4200

cctcccagaa catcacctac cactgcaaga acagcgtggc ctacatggac cagcagactg 4260

gcaacctcaa gaaggccctg ctcctccagg gctccaacga gatcgagatc cgcgccgagg 4320

gcaacagccg cttcacctac agcgtcactg tcgatggctg cacgagtcac accggagcct 4380

ggggcaagac agtgattgaa tacaaaacca ccaagacctc ccgcctgcgc atcatcgatg 4440

tggccccctt ggacgttggt gccccagacc aggaattcgg cttcgacgtt ggccatgtct 4500

gcttcctgta aactccctcc atcccaacct ggctccctcc cacccaacca actttccccc 4560

caacccggaa acagacaagc aacccaaact gaaccccctc aaaagccaaa aaatgggaga 4620

caatttcaca tggactttgg aaaatatttt tttcctttgc attcatctct caaacttagt 4680

ttttatcttt gaccaaccga acatgaccaa aaaccaaaag tgcattcaac cttaccaaaa 4740

aaaaaaaaaa aa 4752

<210> 4
<211> 4572
<212> DNA
<213> Homo sapiens collagen, type I, alpha 2 (hCT1A2)

<400> 4
gtttcggcta agttggaggt actggccacg actgcatgcc cgcgcccgcc aggtgatacc 60

tccgccggtg acccaggggc tctgcgacac aaggagtctg catgtctaag tgctagacat 120

gctcagcttt gtggatacgc ggactttgtt gctgcttgca gtaaccttat gcctagcaac 180

atgccaatct ttacaagagg aaactgtaag aaagggccca gccggagata gaggaccacg 240

tggagaaagg ggtccaccag gccccccagg cagagatggt gaagatggtc ccacaggccc 300

tcctggtcca cctggtcctc ctggcccccc tggtctcggt gggaactttg ctgctcagta 360

tgatggaaaa ggagttggac ttggccctgg accaatgggc ttaatgggac ctagaggccc 420

acctggtgca gctggagccc caggccctca aggtttccaa ggacctgctg gtgagcctgg 480

tgaacctggt caaactggtc ctgcaggtgc tcgtggtcca gctggccctc ctggcaaggc 540

tggtgaagat ggtcaccctg gaaaacccgg acgacctggt gagagaggag ttgttggacc 600

acagggtgct cgtggtttcc ctggaactcc tggacttcct ggcttcaaag gcattagggg 660

acacaatggt ctggatggat tgaagggaca gcccggtgct cctggtgtga agggtgaacc 720

tggtgcccct ggtgaaaatg gaactccagg tcaaacagga gcccgtgggc ttcctggtga 780

gagaggacgt gttggtgccc ctggcccagc tggtgcccgt ggcagtgatg gaagtgtggg 840

tcccgtgggt cctgctggtc ccattgggtc tgctggccct ccaggcttcc caggtgcccc 900

tggccccaag ggtgaaattg gagctgttgg taacgctggt cctgctggtc ccgccggtcc 960

ccgtggtgaa gtgggtcttc caggcctctc cggccccgtt ggacctcctg gtaatcctgg 1020

agcaaacggc cttactggtg ccaagggtgc tgctggcctt cccggcgttg ctggggctcc 1080

cggcctccct ggaccccgcg gtattcctgg ccctgttggt gctgccggtg ctactggtgc 1140

cagaggactt gttggtgagc ctggtccagc tggctccaaa ggagagagcg gtaacaaggg 1200

tgagcccggc tctgctgggc cccaaggtcc tcctggtccc agtggtgaag aaggaaagag 1260

aggccctaat ggggaagctg gatctgccgg ccctccagga cctcctgggc tgagaggtag 1320

tcctggttct cgtggtcttc ctggagctga tggcagagct ggcgtcatgg gccctcctgg 1380

tagtcgtggt gcaagtggcc ctgctggagt ccgaggacct aatggagatg ctggtcgccc 1440

tggggagcct ggtctcatgg gacccagagg tcttcctggt tcccctggaa atatcggccc 1500

cgctggaaaa gaaggtcctg tcggcctccc tggcatcgac ggcaggcctg gcccaattgg 1560

cccagctgga gcaagaggag agcctggcaa cattggattc cctggaccca aaggccccac 1620

tggtgatcct ggcaaaaacg gtgataaagg tcatgctggt cttgctggtg ctcggggtgc 1680

tccaggtcct gatggaaaca atggtgctca gggacctcct ggaccacagg gtgttcaagg 1740

tggaaaaggt gaacagggtc ccgctggtcc tccaggcttc cagggtctgc ctggcccctc 1800

aggtcccgct ggtgaagttg gcaaaccagg agaaaggggt ctccatggtg agtttggtct 1860

ccctggtcct gctggtccaa gaggggaacg cggtccccca ggtgagagtg gtgctgccgg 1920

tcctactggt cctattggaa gccgaggtcc ttctggaccc ccagggcctg atggaaacaa 1980

gggtgaacct ggtgtggttg gtgctgtggg cactgctggt ccatctggtc ctagtggact 2040

cccaggagag aggggtgctg ctggcatacc tggaggcaag ggagaaaagg gtgaacctgg 2100

tctcagaggt gaaattggta accctggcag agatggtgct cgtggtgctc ctggtgctgt 2160

aggtgcccct ggtcctgctg gagccacagg tgaccggggc gaagctgggg ctgctggtcc 2220

tgctggtcct gctggtcctc ggggaagccc tggtgaacgt ggtgaggtcg gtcctgctgg 2280

ccccaatgga tttgctggtc ctgctggtgc tgctggtcaa cctggtgcta aaggagaaag 2340

aggagccaaa gggcctaagg gtgaaaacgg tgttgttggt cccacaggcc ccgttggagc 2400

tgctggccca gctggtccaa atggtccccc cggtcctgct ggaagtcgtg gtgatggagg 2460

cccccctggt atgactggtt tccctggtgc tgctggacgg actggtcccc caggaccctc 2520

tggtatttct ggccctcctg gtccccctgg tcctgctggg aaagaagggc ttcgtggtcc 2580

tcgtggtgac caaggtccag ttggccgaac tggagaagta ggtgcagttg gtccccctgg 2640

cttcgctggt gagaagggtc cctctggaga ggctggtact gctggacctc ctggcactcc 2700

aggtcctcag ggtcttcttg gtgctcctgg tattctgggt ctccctggct cgagaggtga 2760

acgtggtcta ccaggtgttg ctggtgctgt gggtgaacct ggtcctcttg gcattgccgg 2820

ccctcctggg gcccgtggtc ctcctggtgc tgtgggtagt cctggagtca acggtgctcc 2880

tggtgaagct ggtcgtgatg gcaaccctgg gaacgatggt cccccaggtc gcgatggtca 2940

acccggacac aagggagagc gcggttaccc tggcaatatt ggtcccgttg gtgctgcagg 3000

tgcacctggt cctcatggcc ccgtgggtcc tgctggcaaa catggaaacc gtggtgaaac 3060

tggtccttct ggtcctgttg gtcctgctgg tgctgttggc ccaagaggtc ctagtggccc 3120

acaaggcatt cgtggcgata agggagagcc cggtgaaaag gggcccagag gtcttcctgg 3180

cttaaaggga cacaatggat tgcaaggtct gcctggtatc gctggtcacc atggtgatca 3240

aggtgctcct ggctccgtgg gtcctgctgg tcctaggggc cctgctggtc cttctggccc 3300

tgctggaaaa gatggtcgca ctggacatcc tggtacagtt ggacctgctg gcattcgagg 3360

ccctcagggt caccaaggcc ctgctggccc ccctggtccc cctggccctc ctggacctcc 3420

aggtgtaagc ggtggtggtt atgactttgg ttacgatgga gacttctaca gggctgacca 3480

gcctcgctca gcaccttctc tcagacccaa ggactatgaa gttgatgcta ctctgaagtc 3540

tctcaacaac cagattgaga cccttcttac tcctgaaggc tctagaaaga acccagctcg 3600

cacatgccgt gacttgagac tcagccaccc agagtggagc agtggttact actggattga 3660

ccctaaccaa ggatgcacta tggatgctat caaagtatac tgtgatttct ctactggcga 3720

aacctgtatc cgggcccaac ctgaaaacat cccagccaag aactggtata ggagctccaa 3780

ggacaagaaa cacgtctggc taggagaaac tatcaatgct ggcagccagt ttgaatataa 3840

tgtagaagga gtgacttcca aggaaatggc tacccaactt gccttcatgc gcctgctggc 3900

caactatgcc tctcagaaca tcacctacca ctgcaagaac agcattgcat acatggatga 3960

ggagactggc aacctgaaaa aggctgtcat tctacagggc tctaatgatg ttgaacttgt 4020

tgctgagggc aacagcaggt tcacttacac tgttcttgta gatggctgct ctaaaaagac 4080

aaatgaatgg ggaaagacaa tcattgaata caaaacaaat aagccatcac gcctgccctt 4140

ccttgatatt gcacctttgg acatcggtgg tgctgaccag gaattctttg tggacattgg 4200

cccagtctgt ttcaaataaa tgaactcaat ctaaattaaa aaagaaagaa atttgaaaaa 4260

actttctctt tgccatttct tcttcttctt ttttaactga aagctgaatc cttccatttc 4320

ttctgcacat ctacttgctt aaattgtggg caaaagagaa aaagaaggat tgatcagagc 4380

attgtgcaat acagtttcat taactccttc cctcgctccc ccaaaaattt gaattttttt 4440

ttcaacactc ttacacctgt tatggaaaat gtcaaccttt gtaagaaaac caaaataaaa 4500

attgaaaaat aaaaaccata aacatttgca aaaaaaaaaa aaaaaaaaaa aaaaaaaaaa 4560

aaaaaaaaaa aa 4572

<210> 5
<211> 4937
<212> DNA
<213> Homo sapiens collagen, type II, alpha 1 (hCT2A1)

<400> 5
ccatgattcg cctcggggct ccccagacgc tggtgctgct gacgctgctc gtcgccgctg 60

tccttcggtg tcagggccag gatgtccagg aggctggcag ctgtgtgcag gatgggcaga 120

ggtataatga taaggatgtg tggaagccgg agccctgccg gatctgtgtc tgtgacactg 180

ggactgtcct ctgcgacgac ataatctgtg aagacgtgaa agactgcctc agccctgaga 240

tccccttcgg agagtgctgc cccatctgcc caactgacct cgccactgcc agtgggcaac 300

caggaccaaa gggacagaaa ggagaacctg gagacatcaa ggatattgta ggacccaaag 360

gacctcctgg gcctcaggga cctgcagggg aacaaggacc cagaggggat cgtggtgaca 420

aaggtgaaaa aggtgcccct ggacctcgtg gcagagatgg agaacctggg acccctggaa 480

atcctggccc ccctggtcct cccggccccc ctggtccccc tggtcttggt ggaaactttg 540

ctgcccagat ggctggagga tttgatgaaa aggctggtgg cgcccagttg ggagtaatgc 600

aaggaccaat gggccccatg ggacctcgag gacctccagg ccctgcaggt gctcctgggc 660

ctcaaggatt tcaaggcaat cctggtgaac ctggtgaacc tggtgtctct ggtcccatgg 720

gtccccgtgg tcctcctggt ccccctggaa agcctggtga tgatggtgaa gctggaaaac 780

ctggaaaagc tggtgaaagg ggtccgcctg gtcctcaggg tgctcgtggt ttcccaggaa 840

ccccaggcct tcctggtgtc aaaggtcaca gaggttatcc aggcctggac ggtgctaagg 900

gagaggcggg tgctcctggt gtgaagggtg agagtggttc cccgggtgag aacggatctc 960

cgggcccaat gggtcctcgt ggcctgcctg gtgaaagagg acggactggc cctgctggcg 1020

ctgcgggtgc ccgaggcaac gatggtcagc caggccccgc agggcctccg ggtcctgtcg 1080

gtcctgctgg tggtcctggc ttccctggtg ctcctggagc caagggtgaa gccggcccca 1140

ctggtgcccg tggtcctgaa ggtgctcaag gtcctcgcgg tgaacctggt actcctgggt 1200

cccctgggcc tgctggtgcc tccggtaacc ctggaacaga tggaattcct ggagccaaag 1260

gatctgctgg tgctcctggc attgctggtg ctcctggctt ccctgggcca cggggccctc 1320

ctggccctca aggtgcaact ggtcctctgg gcccgaaagg tcagacgggt gaacctggta 1380

ttgctggctt caaaggtgaa caaggcccca agggagaacc tggccctgct ggcccccagg 1440

gagcccctgg acccgctggt gaagaaggca agagaggtgc ccgtggagag cctggtggcg 1500

ttgggcccat cggtccccct ggagaaagag gtgctcccgg caaccgcggt ttcccaggtc 1560

aagatggtct ggcaggtccc aagggagccc ctggagagcg agggcccagt ggtcttgctg 1620

gccccaaggg agccaacggt gaccctggcc gtcctggaga acctggcctt cctggagccc 1680

ggggtctcac tggccgccct ggtgatgctg gtcctcaagg caaagttggc ccttctggag 1740

cccctggtga agatggtcgt cctggacctc caggtcctca gggggctcgt gggcagcctg 1800

gtgtcatggg tttccctggc cccaaaggtg ccaacggtga gcctggcaaa gctggtgaga 1860

agggactgcc tggtgctcct ggtctgaggg gtcttcctgg caaagatggt gagacaggtg 1920

ctgcaggacc ccctggccct gctggacctg ctggtgaacg aggcgagcag ggtgctcctg 1980

ggccatctgg gttccaggga cttcctggcc ctcctggtcc cccaggtgaa ggtggaaaac 2040

caggtgacca gggtgttccc ggtgaagctg gagcccctgg cctcgtgggt cccaggggtg 2100

aacgaggttt cccaggtgaa cgtggctctc ccggtgccca gggcctccag ggtccccgtg 2160

gcctccccgg cactcctggc actgatggtc ccaaaggtgc atctggccca gcaggccccc 2220

ctggggctca gggccctcca ggtcttcagg gaatgcctgg cgagagggga gcagctggta 2280

tcgctgggcc caaaggcgac aggggtgacg ttggtgagaa aggccctgag ggagcccctg 2340

gaaaggatgg tggacgaggc ctgacaggtc ccattggccc ccctggccca gctggtgcta 2400

atggcgagaa gggagaagtt ggacctcctg gtcctgcagg aagtgctggt gctcgtggcg 2460

ctccgggtga acgtggagag actgggcccc ccggaccagc gggatttgct gggcctcctg 2520

gtgctgatgg ccagcctggg gccaagggtg agcaaggaga ggccggccag aaaggcgatg 2580

ctggtgcccc tggtcctcag ggcccctctg gagcacctgg gcctcagggt cctactggag 2640

tgactggtcc taaaggagcc cgaggtgccc aaggcccccc gggagccact ggattccctg 2700

gagctgctgg ccgcgttgga cccccaggct ccaatggcaa ccctggaccc cctggtcccc 2760

ctggtccttc tggaaaagat ggtcccaaag gtgctcgagg agacagcggc ccccctggcc 2820

gagctggtga acccggcctc caaggtcctg ctggaccccc tggcgagaag ggagagcctg 2880

gagatgacgg tccctctggt gccgaaggtc caccaggtcc ccagggtctg gctggtcaga 2940

gaggcatcgt cggtctgcct gggcaacgtg gtgagagagg attccctggc ttgcctggcc 3000

cgtcgggtga gcccggcaag cagggtgctc ctggagcatc tggagacaga ggtcctcctg 3060

gccccgtggg tcctcctggc ctgacgggtc ctgcaggtga acctggacga gagggaagcc 3120

ccggtgctga tggcccccct ggcagagatg gcgctgctgg agtcaagggt gatcgtggtg 3180

agactggtgc tgtgggagct cctggagccc ctgggccccc tggctcccct ggccccgctg 3240

gtccaactgg caagcaagga gacagaggag aagctggtgc acaaggcccc atgggaccct 3300

caggaccagc tggagcccgg ggaatccagg gtcctcaagg ccccagaggt gacaaaggag 3360

aggctggaga gcctggcgag agaggcctga agggacaccg tggcttcact ggtctgcagg 3420

gtctgcccgg ccctcctggt ccttctggag accaaggtgc ttctggtcct gctggtcctt 3480

ctggccctag aggtcctcct ggccccgtcg gtccctctgg caaagatggt gctaatggaa 3540

tccctggccc cattgggcct cctggtcccc gtggacgatc aggcgaaacc ggccctgctg 3600

gtcctcctgg aaatcctgga ccccctggtc ctccaggtcc ccctggccct ggcatcgaca 3660

tgtccgcctt tgctggctta ggcccgagag agaagggccc cgaccccctg cagtacatgc 3720

gggccgacca ggcagccggt ggcctgagac agcatgacgc cgaggtggat gccacactca 3780

agtccctcaa caaccagatt gagagcatcc gcagccccga gggctcccgc aagaaccctg 3840

ctcgcacctg cagagacctg aaactctgcc accctgagtg gaagagtgga gactactgga 3900

ttgaccccaa ccaaggctgc accttggacg ccatgaaggt tttctgcaac atggagactg 3960

gcgagacttg cgtctacccc aatccagcaa acgttcccaa gaagaactgg tggagcagca 4020

agagcaagga gaagaaacac atctggtttg gagaaaccat caatggtggc ttccatttca 4080

gctatggaga tgacaatctg gctcccaaca ctgccaacgt ccagatgacc ttcctacgcc 4140

tgctgtccac ggaaggctcc cagaacatca cctaccactg caagaacagc attgcctatc 4200

tggacgaagc agctggcaac ctcaagaagg ccctgctcat ccagggctcc aatgacgtgg 4260

agatccgggc agagggcaat agcaggttca cgtacactgc cctgaaggat ggctgcacga 4320

aacataccgg taagtggggc aagactgtta tcgagtaccg gtcacagaag acctcacgcc 4380

tccccatcat tgacattgca cccatggaca taggagggcc cgagcaggaa ttcggtgtgg 4440

acatagggcc ggtctgcttc ttgtaaaaac ctgaacccag aaacaacaca atccgttgca 4500

aacccaaagg acccaagtac tttccaatct cagtcactct aggactctgc actgaatggc 4560

tgacctgacc tgatgtccat tcatcccacc ctctcacagt tcggactttt ctcccctctc 4620

tttctaagag acctgaactg ggcagactgc aaaataaaat ctcggtgttc tatttattta 4680

ttgtcttcct gtaagacctt cgggtcaagg cagaggcagg aaactaactg gtgtgagtca 4740

aatgccccct gagtgactgc ccccagccca ggccagaaga cctcccttca ggtgccgggc 4800

gcaggaactg tgtgtgtcct acacaatggt gctattctgt gtcaaacacc tctgtatttt 4860

ttaaaacatc aattgatatt aaaaatgaaa agattattgg aaagtaaaaa aaaaaaaaaa 4920

aaaaaaaaaa aaaaaaa 4937

<210> 6
<211> 28

<212>  DNA
<213>  Artificial Sequence

<220>
<223>  forward primer for K4 promoter-ApaI

<400>  6
gggcccggtt cgacattgga ttttggtc                                          28


<210>  7
<211>  28
<212>  DNA
<213>  Artificial Sequence

<220>
<223>  reverse primer for K4 promoter-BamHI

<400>  7
ggatccgatg cctgtgtctt tgagttgc                                          28


<210>  8
<211>  33
<212>  DNA
<213>  Artificial Sequence

<220>
<223>  forward primer for collagen type 1 alpha 1- AgeI

<400>  8
accggtacca tgttcagctt tgtggacctc cgg                                    33


<210>  9
<211>  29
<212>  DNA
<213>  Artificial Sequence

<220>
<223>  reverse primer for collagen type 1 alpha 1-SalI

<400>  9
gtcgacttac aggaagcaga catggccaa                                         29


<210>  10
<211>  37
<212>  DNA
<213>  Artificial Sequence

<220>
<223>  forward primer for collagen type 1 alpha 1-Bgl2-Nhe1

<400>  10
agatctgcta gcaccatgtt cagctttgtg gacctcc                                37


<210>  11
<211>  27
<212>  DNA
<213>  Artificial Sequence

<220>
<223>  reverse primer for collagen type 1 alpha 1- Sal1

<400>  11
gtcgacttac aggaagcaga catggcc                                          27


<210>  12
<211>  37
<212>  DNA
<213>  Artificial Sequence

<220>
<223>  forward primer for collagen type 1 alpha2- BamH1-Nhe1

<400>  12
ggatccgcta gcaccatgct cagctttgtg gatacgc                               37


<210>  13
<211>  33
<212>  DNA
<213>  Artificial Sequence

<220>
<223>  reverse primer for collagen type 1 alpha2- Sal1-Cla1

<400>  13
gtcgacatcg atttatttga aacagactgg gcc                                   33


<210>  14
<211>  27
<212>  DNA
<213>  Artificial Sequence

<220>
<223>  forward primer for collagen type 2 alpha1- EcoRI

<400>  14
gaattcatga ttcgcctcgg ggctccc                                          27


<210>  15
<211>  27
<212>  DNA
<213>  Artificial Sequence

<220>
<223>  reverse primer for collagen type 2 alpha1- SolI

<400>  15
gtcgacttac aagaagcaga ccggccc                                          27


<210>  16
<211>  35
<212>  DNA
<213>  Artificial Sequence

<220>
<223>  forward primer for collagen type 2 alpha1- BamH1-Nhe1

<400>  16
ggatccgcta gcaccatgat tcgcctcggg gctcc                                35

<210>  17
<211>  33
<212>  DNA
<213>  Artificial Sequence

<220>
<223>  reverse primer for collagen type 2 alpha1- Sal1-Cla1

<400>  17
gtcgacatcg atttacaaga agcagaccgg ccc                                  33